## Europäisches Patentamt
## European Patent Office
## Office européen des brevets

(19)

(11) Numéro de publication: **0 032 409**
**B1**

(12) # FASCICULE DE BREVET EUROPEEN

(45) Date de publication du fascicule du brevet:
**17.08.83**

(51) Int. Cl.³: **A 23 K 1/175,** A 23 K 1/18,
A 61 K 33/40

(21) Numéro de dépôt: **81200003.2**

(22) Date de dépôt: **05.01.81**

(54) Compositions à ingérer par les ruminants, procédé pour leur préparation.

(30) Priorité: **11.01.80 FR 8000774**

(43) Date de publication de la demande:
**22.07.81 Bulletin 81/29**

(45) Mention de la délivrance du brevet:
**17.08.83 Bulletin 83/33**

(84) Etats contractants désignés:
**AT BE CH DE FR GB IT LI NL SE**

(56) Documents cités:
**FR-A-2 319 304**
**FR-A-2 345 940**
**chemical abstracts, vol. 89, no. 3, 17 juillet 1978,**
**page 538, abrégé 22747p, Columbus, Ohio, US**
**CHEMICAL ABSTRACTS, vol. 45, no. 5, 10 mars**
**1950, 2148e Columbus, Ohio, US, L. W. HAASE:**
**»Bactericidal action of hydrogen peroxide, perox-**
**ides, and compounds liberating oxygen«**

(73) Titulaire: **INTEROX Société anonyme dite:, Rue du**
**Prince Albert, 33, B-1050 Bruxelles (BE)**

(72) Inventeur: **Gago, Ignace, Chaussée de Nivelles, 53,**
**B-1420 Braine-l'Alleud (BE)**
Inventeur: **Coppens, Guillaume, Avenue Louis**
**Jasmin, 89, B-1150 Bruxelles (BE)**

(74) Mandataire: **Meyers, Liliane et al, Solvay & Cie**
**Département de la propriété industrielle 310, rue de**
**Ransbeek, B-1120 Bruxelles (BE)**

## Compositions à ingérer par les ruminants, procédé pour leur préparation

L'invention concerne des compositions à ingérer par les ruminants telles que des compositions alimentaires ou des compositions à usage vétérinaire ou prophylactique. Elle concerne également un procédé pour la préparation de telles compositions.

Les ruminants sont capables de synthétiser les acides aminés essentiels à l'édification des protéines de l'organisme au départ de glucides, tels que la cellulose, les hémicelluloses, l'amidon et les sucres solubles, et de substances alimentaires azotées non protéiques, telles que l'urée et certains sels ammoniacaux. Lors de la digestion, les glucides sont en grande partie dégradés dans le rumen par divers types d'enzymes bactériennes spécifques pour donner des produits intermédiaires (cellobiose, maltose, glucose, xylose, acides uroniques, etc° dont la nature est fonction de celle des glucides de départ. Ces produits sont ensuite transformés par d'autres bactéries en produits terminaux dont les acides gras volatils à chaînes courtes tels que l'acide acétique. L'apport énergétique des glucides permet notamment à la flore du rumen de fabriquer des protéines microbiennes, entre autres avec l'ammoniaque libéré par hydroylse bactérienne des substances azotées.

La transformation des glucides s'accompagne de la formation de déchets tels que le méthane. Des proportions anormalement élevées de ce produit conduisent à des pertes énergétiques élevées.

Lorsqu'il y a une contamination du rumen par des protozoaires, qui peut, par exemple, résulter de l'ingestion d'eau de boisson ou de fourrages contaminés ou d'un contact avec des animaux infectés, on observe une réduction de la formation d'acide acétique dans le rumen.

En outre, on observe également une réduction de l'efficacité de la synthèse des protéines bactériennes dans le rumen. Pour compenser cette réduction du rendement, on est contraint de compléter l'alimentation des ruminants au moyen de substances protéiques telles que des tourteaux de graines oléagineuses, des céréales et des farines alimentaires dont une partie pourrait servir à l'alimentation humaine directe. Ceci va à l'encontre de tous les efforts faits pour accroître les ressources alimentaires humaines.

La destruction des protozoaires présents dans le rumen, en vue d'améliorer la synthèse des protéines, est par ailleurs très malaisée. En effet, il faut sélectionner pour ce faire un agent défaunant capable de détruire les protozoaires présents dans le rumen sans pour autant détruire les autres microorganismes nécessaires à la digestion normale. Il faut en outre que cet agent défaunant ne soit pas toxique pour l'animal.

On a déjà proposé dans le certificat d'auteur SU-A-600 995 (ALL-UNION SCIENTIFIC-RESEARCH AND TECHNOLOGICAL INSTITUTE OF POULTRY) de stimuler la croissance de poulets en leur faisant ingurgiter une composition contenant du peroxyde de calcium et de la farine d'os.

Par ailleurs, il est connu par la demande de brevet FR-A-2 345 940 (ASTRA EWOS AB) d'administrer à des animaux des compositions antibactériennes contenant, à titre de constituants essentiels, un composé peroxydé, de la lactoperoxydase et un thiocyanate.

Il est également connu par la demande de brevet FR-A-2 319 304 (FARMOS OY) d'abaisser le risque de contamination du lait par le fumier en réduisant le développement de certaines bactéries (les clostridions), dans le tractus digestif des animaux. Pour cela, on leur administre une composition contenant du chlorate de potassium, du nitrate de sodium, du peroxyde de benzoyle et du lactose.

Selon l'article de L. W. Haase (Pharmazie, 1950, 5, p. 436−437; Chemical Abstracts, 1950, 45 (5), colonne 2148e), les peroxydes sont peu actifs contre les bactéries telles que Escherichia coli et Bacillus prodigiosus et sont inefficaces comme désinfectants oraux.

La présente invention vise à procurer des compositions à ingérer par les ruminants qui présentent un effet défaunant vis-à-vis des protozoaires, qui ne détruisent pas les microorganismes présents dans le rumen et nécessaires à la digestion, qui conduisent à une amélioration de l'assimilation par les ruminants des matières azotées non protéiques et des glucides ingérés, et qui, enfin, ne sont pas toxiques. Ces compositions permettent d'alimenter les ruminants au moyen de rations contenant plus de matières azotées non protéiques et moins de protéines ou d'augmenter la production. De plus, elles réduisent la méthanogénèse dans le rumen.

L'invention concerne à cet effet des compositions à ingérer par les ruminants qui contiennent un peroxyde d'un métal du Groupe 2 du Tableau périodique.

Le peroxyde de métal du Groupe 2 est en général choisi parmi les peroxydes de calcium, de magnésium, de zinc, ou de strontium et leurs mélanges. Les peroxydes de calcium et de magnésium sont particulièrement préférés. De bons résultats ont été obtenus avec le peroxyde de calcium.

Le peroxyde de métal du Groupe 2 mis en oeuvre peut être de toute qualité connue. Quand il s'agit du peroxyde de calcium, on utilise en général un produit commercial contenant de 30 à 90% de peroxyde de calcium, le solde étant constitué notamment d'oxyde et d'hydroxyde de calcium et éventuellement d'un sel de calcium et d'eau. D'autres qualités de peroxyde de calcium peuvent également convenir.

Le peroxyde de métal du Groupe 2 peut être mis en oeuvre en quantités fort variables. En général, il est introduit dans les compositions selon l'invention en proportions telles que la ration ingérée quotidiennement, calculée en oxygène actif par rapport au poids de l'animal soit supérieure à

0 032 409

0,00005% et le plus souvent supérieure à 0,0001%. En général, ces proportions sont telles que la ration de peroxyde ingérée quotidiennement ne dépasse pas 0,06% et le plus souvent 0,04% (claculée en oxygène actif).

De préférence, on introduit le peroxyde dans les compositions selon l'invention en proportions telles que la ration ingérée en une prise par l'animal, soit comprise entre 0,0001 et 0,1% en poids de composé peroxydé tel quel par rapport au poids de l'animal. De bons résultats ont été obtenus avec des ingestions correspondant à 0,001 à 0,05% en poids de peroxyde par rapport au poids de l'animal. D'autres doses peuvent également convenir.

Le peroxyde peut être administré en une prise unique ou en plusieurs prises périodiques. En général, pour des raisons de commodités et pour éviter une nouvelle contamination ultérieure, on l'administre de façon périodique. La périodicité peut être variable et est fonction des risques de contamination, du mode d'alimentation, de la quantité de peroxyde administrée par ration, des autres constituants de la composition et de sa présentation, des animaux eux-mêmes (âge, sexe, état physiologique, race), etc. De bons résultats ont été obtenus en administrant quotidiennement des rations de la compositon selon l'invention.

Les compositions selon l'invention peuvent se présenter sous différentes formes. Elles peuvent ainsi se trover sous la forme de compositions alimentaires contenant le peroxyde et un ou plusieurs aliments pour ruminants tels que des fourrages de tous types et sous toutes leurs formes (verts, déshydratés, agglomérés, etc) comme l'herbe et les autres graminées fourragères, les céréales fourragères (orge, mais, avoine, blé, sorgho, soja, seigle), les légumineuses (féverole, luzerne, sainfoin, trèfles), les racines, tubercules et leurs sous produits (betteraves et pulpe de betteraves), le choux, le colza, le torunesol, les déchets de végétaux (fanes, rafles, balles de céréales, son, épis de maïs égrénés, bagasse) et les fécules, ainsi que des tourteaux de graines oléagineuses, des sirops, et des matières alimentaires azotées telles que l'urée et ses dérivés (biuret, uréides) et les sels ammoniacaux. De telles compositions alimentaires peuvent en outre contenir de petites quantités d'autres additifs tels que des sels minéraux, des vitamines, des oligoéléments, des graisses, des produits aromatisants et des liants. Lorsque les ruminants sont alimentés essentiellement par des compositions alimentaires, la proportion de peroxyde dans cette composition est en général comprise entre 0,05 et 20% et le plus souvent entre 0,1 et 10% du poids de matières sèches ingérées. De bons résultats ont été obtenus en utilisant des compositions alimentaires contenant entre 0,2 et 5% en poids de peroxyde. Si les animaux ont d'autres ressources alimentaires, la quantité de peroxyde dans la composition alimentaire peut être augmentée en proportion.

Les compositions alimentaires selon l'invention peuvent se présenter sous différentes formes physiques par exemple sous formes compactées, granulées, en poudre ou même semi-liquides.

Les compositions selon l'invention peuvent également se présenter sous forme de complément à l'alimentation normale. On peut ainsi les présenter sous forme de compositions prophylactiques ou vétérinaires contenant le peroxyde, éventuellement un support de type glucidique (matières amylacées, cellulosiques, etc) digestible par les animaux, et divers autres additifs tels que des vitamines, des sels minéraux, des oligo-éléments, des émulsionnants, des produits aromatisants et des liants ainsi que certaines substances actives adaptées aux besoins spécifiques de l'animal. Ces suppléments peuvent se présenter sous différentes formes physiques (poudres, solides compactés ou granulés ou éventuellement semi-liquides) et peuvent être donnés aux animaux sous forme de ration distincte des aliments. On peut également de façon avantageuse les mélanger au moment de l'emploi aux rations alimentaires. De telles compositions vétérinaires peuvent contenir de 5 à 90% et de préférence de 10 à 60% en poids de peroxyde.

Les compositions selon l'invention peuvent également se présenter sous forme de pierres dites à lécher (ou à grignoter). Dans ce cas outre le peroxyde, elles peuvent contenir divers autres additifs tels que des vitamines, plus particulièrement des vitamines A, D, E et PP, des oligo-éléments (fer, cuivre, cobalt, manganèse, zinc, sélénium, étain, etc), des matières azotées non protéiques (urée, etc), des sels minéraux (chlorures, fluorures, iodures, carbonates, bicarbonates, phosphates de tous types, neutres ou acides de métaux des groupes 1 et 2 du Tableau périodique), des matières soufrées (soufre, sulfures), des supports glucidiques (contenant des matières amylacées ou cellulosiques telles que les farines et les sons) tels que ceux cités ci-avant en tant qu'aliments, des produits aromatisants, des graisses (graisse de suif, graisse de coco) et divers autres additifs habituellement utilisés dans les pierres à lécher ou à grignoter pouvant avoir notamment un effet de liant ou de charge (sulfate, oxyde et hydroxyde de magnésium, ciments, gypse, chaux, sulfate d'aluminium, alun, gélatine).

Les compositions selon l'invention se trouvant sous forme de pierres à lécher ou à grignoter peuvent contenir des quantités variables de peroxyde. Elles contiennent en général de 2 à 60% et de préférence de 5 à 50% en poids de peroxyde.

Ainsi, des compositions selon l'invention pour pieeres à lécher convenant bien contiennent:
— de 2 à 60% en poids de peroxyde,
— de 0,1 à 40% en poids de sels minéraux,
— de 0,1 à 60% en poids de matières azotées,
— de 0,1 à 20% en poids de supports glucidiques,
— de 0,1 à 10% en poids d'un mélange à base d'oligoéléments et de vitamines,

3

— de 0,5 à 30% en poids de produits aromatisants,
— de 0 à 5% en poids de graisses,
— de 0 à 5% en poids de matières sulfurées, et
— de 0 à 10% en poids d'autres additifs habituellement utilisés dans les pierres à lécher ou à grignoter.

L'invention concerne également un procédé pour la préparation de compositions alimentaires à ingérer par les ruminants se présentant sous forme de pierres à lécher selon lequel

a) on prépare un mélange homogène sec contenant un peroxyde de métal du groupe 2 du Tableau périodique et au moins un autre constituant insoluble ou peu soluble dans l'eau choisi parmi les supports glucidiques, les graisses, les mélanges à base d'oligoéléments et de vitamines, les matières sulfurées ainsi que les sels minéraux, matières azotées et autres additifs insolubles ou peu solubles dans l'eau;

b) on prépare une solution aqueuse contenant au moins un constituant choisi parmi les produits aromatisants ainsi que les sels minéraux, matières azotées et autres additifs solubles dans l'eau;

c) on ajoute au mélange homogène sec la solution aqueuse, en pétrissant, de manière à obtenir une pâte; et

d) on fait sécher la pâte.

Par constituants insolubles ou peu solubles dans l'eau, on entend désigner les constituants dont la solubilité dans l'eau est inférieure à la dose mise en oeuvre rapportée à la quantité d'eau mise en oeuvre pour fabriquer la pierre à lécher. La quantité d'eau mise en oeuvre pour la préparation de la solution aqueuse peut varier dans de larges limites. En général, pour réduire les opérations de séchage, on préfère utiliser des quantités d'eau ne dépassant pas le poids sec de pierres à lécher ou à grignoter produites et de préférence ne dépassant pas 50% de ce poids.

La pâte obtenue par pétrissage du mélange homogène sec et de la solution aqueuse peut éventuellement être moulée avant d'être séchée.

Le séchage peut se faire selon toute technique connue en elle-même, par exemple par séchage à l'air à la température ambiante, par séchage sous circulation forcée d'air à des températures variables, en général comprises entre la température ambiante et 80°C.

Un procédé avantageux consiste à:

a) mélanger de manière homogène à sec le peroxyde avec les supports glucidiques, le mélange à base d'oligoéléments et de vitamines, les sels minéraux et matières azotées peu solubles ou insolubles dans l'eau, ainsi que les graisses éventuelles, les matières sulfurées éventuelles et les autres additifs peu solubles ou insolubles éventuels,

b) dissoudre dans de l'eau les produits aromatisants, les sels minéraux et matières azotées solubles dans l'eau ainsi que les autres additifs solubles éventuels,

c) ajouter la solution aqueuse au mélange sec en pétrissant pour obtenir une pâte que l'on moule, et

d) sécher la pâte moulée.

Les compositions décrites plus haut peuvent être administrées par voie orale aux ruminants en mélange avec au moins une substance alimentaire.

Un procédé particulier consiste à administrer à l'animal de telles compositions telles que la ration de peroxyde ingérée calculée en oxygène actif par rapport au poids de l'animal varie de 0,00005 à 0,06% et de préférence de 0,0001 à 0,04%.

Cette composition peut avantageusement être administrée quotidiennement pendant n'importe quelle période et même pour la durée totale de la vie de l'animal.

Un procédé particulièrement avantageux consiste à administrer à l'animal des compositions choisies de manière que la ration de peroxyde ingérée varie dans le temps. Ainsi, on peut avantageusement, en début de traitement administrer à l'animal des compositions telles que la ration quotidienne de peroxyde calculée en oxygène actif est relativement élevée, en général de 0,0005 à 0,06% du poids de l'animal jusqu'à défaunation et ensuite, de maintenir la défaunation par administration de rations réduites de peroxyde, en général de 0,00005 à 0,002%.

Afin d'illustrer l'invention sans pour autant en limiter la portée, on donne ci-après des exemples pratiques de réalisation.

## Exemple 1

L'effet biologique de l'agent défaunant (peroxyde de calcium) a été déterminé à l'aide d'un mouton fistulé au niveau du rumen.

Le mouton, d'un poids initial d'environ 70 kg, a reçu deux fois par jour une ration alimentaire constituée de 200 g de foin et de 400 g de boulettes de pulpe contenant un équivalent d'environ 14% de protéines brutes (par moitié sous forme d'urée et par moitié sous forme de protéines végétales) par rapport au poids de matière séché. L'essai a duré 138 jours.

A partir du 52e jour d'alimentation, une suspension aqueuse de peroxyde de calcium a été infusée par la fistule du rumen, deux fois par jour, au moment où l'animal consommait sa ration alimentaire.

Les doses quotidiennes totales ont été augmentées progressivement jusqu'au 116ème jour d'alimentation. On a supprimé ensuite toute infusion de peroxyde de calcium. Les doses injectées sont mentionnées au Tableau 1 ci-après.

Tableau 1

| Période | Dose quotidienne de peroxyde de calcium (à 80% en $CaO_2$) injectée, g |
|---|---|
| ler au 87ème jour | 0 |
| 88ème au 94ème jour | 1,26 |
| 95ème au 98ème jour | 3,16 |
| 99ème au 101ème jour | 6,32 |
| 102ème au 106ème jour | 12,64 |
| 107ème au 112ème jour | 18,96 |
| 113ème au 116ème jour | 31,6 |
| 117ème au 138ème jour | 0 |

Pendant l'expérience, on a prélevé régulièrement des échantillons du contenu du rumen (du 36ème au 138ème jour) et on a dosé les gaz dans le rumen (du 44ème au 128ème jour) selon les techniques décrites dans l'article D. Demeyer et C. Van Nevel, Revue de l'Agriculture, 1978, 31 (6), p. 1093 – 1112.

A partir du 108ème jour on a observé une disparition complète des protozoaires.

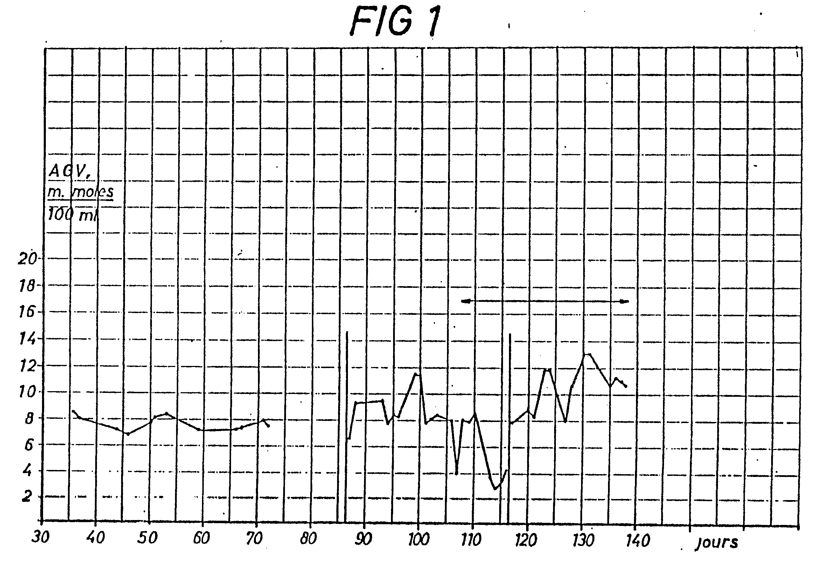

Les acides gras volatils (acide acétique, acide propionique, acide butyrique, acide valérique, acide caproïque) ont été dosés et l'évolution de leur teneur, exprimée en millimoles d'acides gras volatils (AGV) en fonction du temps exprimé en jours, est donnée à la figure 1. L'examen de la figure 1 montre qu'après injection de peroxyde de calcium la teneur moyenne en acides gras volatils a augmenté.

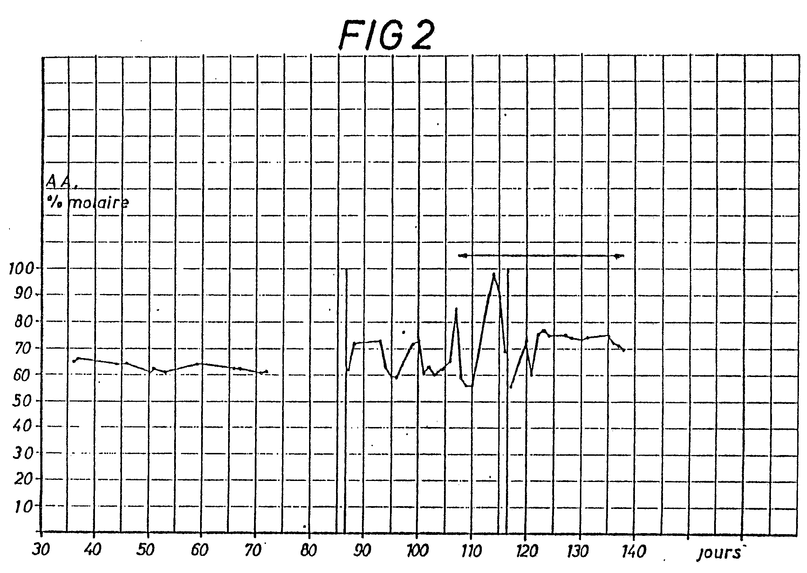

L'évolution de la teneur en acide acétique (AA), exprimée en % molaire des acides gras volatils en fonction du temps exprimé en jours est donnée à la figure 2. L'examen de la figure 2 montre une augmentation du pourcentage moyen d'acide acétique dans les acides gras volatils après injection du peroxyde de calcium.

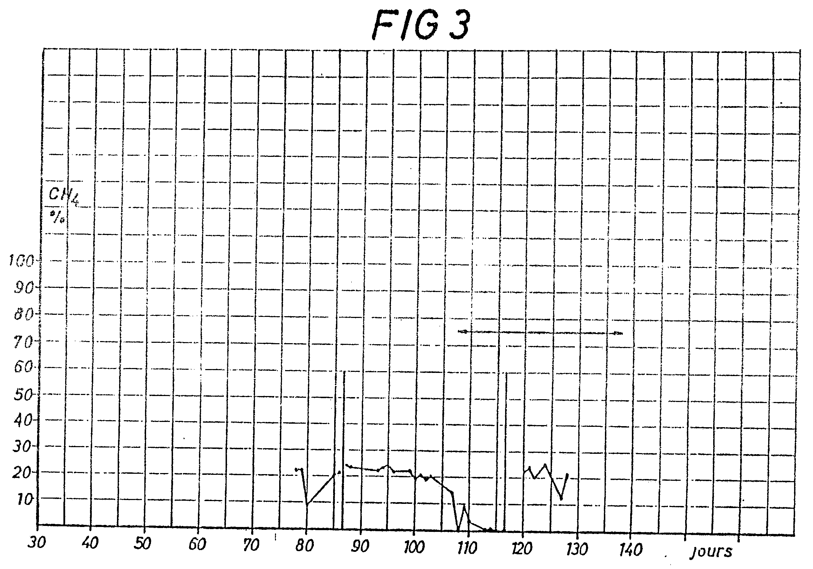

L'évolution de la teneur en méthane ($CH_4$), exprimée en % des gaz dans le rumen, en fonction du temps exprimé en jours est donnée à la figure 3. L'examen de la figure 3 montre une réduction du pourcentage de méthane dans les gaz au cours de l'injection de peroxyde de clacium.

### Exemple 2

On prépare une pierre à grignoter ayant la composition donnée au Tableau 2 ci-après:

Tableau 2

| Constituants | % en poids |
|---|---|
| — peroxyde de calcium à 80% de $CaO_2$ | 12 |
| — phosphate monoacide de calcium | 30 |
| — $Na_2CO_3$ | 1,5 |
| — son de blé | 4 |
| — urée | 20 |
| — mélasse | 22 |
| — NaCl | 2 |
| — farine de blé | 3,5 |
| — mélange d'oligoéléments et de vitamines de marque PROTEXTOMIX de qualité P 22 vendu par PROTECTOR | 5 |

Pour ce faire, on mélange de manière homegène et à sec le peroxyde de calcium, le phosphate monoacide de calcium, le son et la farine de blé et le mélange d'oligoéléments et de vitamines.

On dissout d'autre part à chaud la mélasse dans 3,84% de son poids d'eau ainsi que le chlorure de sodium, et le carbonate de sodium.

On ajoute progressivement la solution au mélange sec en pétrissant jusqu'à obtention d'une pâte. On moule la pâte et on la sèche sous ventilation à 40°C.

Cette pierre à grignoter a été consommée par 3 moutons à raison de 47 à 122 g par jour et par animal. L'appétence était très bonne.

**Revendications**

1. Compositions à ingérer par les ruminants, caractérisées en ce qu'elles contiennent un peroxyde d'un métal du Groupe 2 du Tableau périodique.

2. Compositions selon la revendication 1, caractérisées en ce qu'elles contiennent du peroxyde de magnésium.

3. Compositions selon la revendication 1, caractérisées en ce qu'elles contiennent du peroxyde de calcium.

4. Compositions selon l'une quelconque des revendications 1 à 3, caractérisées en ce qu'elles contiennent en outre au moins un aliment pour ruminants.

5. Compositions selon la revendication 4, caractérisées en ce qu'elles contiennent de 0,2 à 5% en poids de peroxyde.

6. Compositions selon l'une quelconque des revendications 1 à 3, caractérisées en ce qu'elles contiennent en outre au moins un additif choisi parmi les supports glucidiques, les vitamines, les sels minéraux, les oligo-éléments, les émulsionnants, les produits aromatisants, les liants et certaines substances actives adaptées aux besoins spécifiques de l'animal.

7. Compositions selon la revendication 6, caractérisées en ce qu'elles contiennent de 10 à 60% en poids de peroxyde.

8. Compositions selon l'une quelconque des revendications 1 à 3, caractérisées en ce qu'elles se présentent sous forme de pierre à lécher et contiennent en outre au moins un additif choisi parmi les vitamines, les oligo-éléments, les matières azotées non protéiques, les sels minéraux, les matières soufrées, les supports glucidiques, les produits aromatisants et les autres additifs habituellement utilisès dans les pierres à lécher.

9. Compositions selon la revendication 8, caractérisées en ce qu'elles contiennent de 5 à 50% en poids de peroxyde.

10. Procédé pour la préparation de compositions selon la revendication 8 ou 9 caractérisé en ce que:
a) on prépare un mélange homogène sec contenant un peroxyde de métal du groupe 2 du Tableau périodique et au moins un autre constituant insoluble ou peu soluble dans l'eau choisi parmi les supports glucidiques, les graisses, les mélanges à base d'oligo-éléments et de vitamines, les matières sulfurées ainsi que les sels minéraux, matières azotées et autres additifs insolubles ou peu solubles dans l'eau;

b) on prépare une solution aqueuse contenant au moins un constituant choisi parmi les produits aromatisants ainsi que les sels minéraux, matières azotées et autres additifs solubles dans l'eau;

c) on ajoute au mélange homogène sec la solution aqueuse, en pétrissant, de manière à obtenir une pâte; et

d) on fait sécher la pâte.

## Patentansprüche

1. Durch Wiederkäuer einzunehmende Zusammensetzungen, dadurch gekennzeichnet, daß diese ein Peroxyd eines Metalls der Gruppe 2 des Periodischen Systems enthalten.

2. Zusammensetzungen nach Anspruch 1, dadurch gekennzeichnet, daß sie Magnesiumperoxyd enthalten.

3. Zusammensetzungen nach Anspruch 1, dadurch gekennzeichnet, daß sie Calciumperoxyd enthalten.

4. Zusammensetzungen nach einem der Ansprüche 1 bis 3, dadurch gekennzeichnet, daß sie außerdem mindestens ein Nahrungsmittel für Wiederkäuer enthalten.

5. Zusammensetzungen nach Anspruch 4, dadurch gekennzeichnet, daß sie 0,2 bis 5 Gew.-% Peroxyd enthalten.

6. Zusammensetzungen nach einem der Ansprüche 1 bis 3, dadurch gekennzeichnet, daß sie außerdem mindestens einen Zusatzstoff enthalten, ausgewählt unter Kohlenhydrat-Trägerstoffen, Vitaminen, Mineralsalzen, Spurenelementen, Emulgatoren, Aromatisierungsmitteln, Bindemitteln und bestimmten Aktivsubstanzen, angepaßt an die speziellen Bedürfnisse des Tieres.

7. Zusammensetzungen nach Anspruch 6, dadurch gekennzeichnet, daß sie 10 bis 60 Gew.-% Peroxyd enthalten.

8. Zusammensetzungen nach einem der Ansprüche 1 bis 3, dadurch gekennzeichnet, daß sie in Form eines Lecksteins dargeboten sind und außerdem mindestens einen Zusatzstoff enthalten, ausgewählt unter Vitaminen, Spurenelementen, stickstoffhaltigen Nicht-Eiweißsubstanzen, Mineralsalzen, schwefelhaltigen Substanzen, Kohlenhydrat-Trägerstoffen, Aromatisierungsmitteln und anderen Zusatzstoffen, die üblicherweise in Lecksteinen benutzt werden.

9. Zusammensetzungen nach Anspruch 8, dadurch gekennzeichnet, daß sie 5 bis 50 Gew.-% Peroxyd enthalten.

10. Verfahren zur Herstellung der Zusammensetzungen nach Anspruch 8 oder 9, dadurch gekennzeichnet, daß

a) man eine trockene homogene Mischung enthaltend ein Peroxyd eines Metalls der Gruppe 2 des Periodischen Systems und mindestens einen anderen in Wasser unlöslichen oder wenig löslichen Bestandteil ausgewählt unter den Kohlenhydrat-Trägerstoffen, Fetten, Spurenelement- und Vitaminmischungen, schwefelhaltigen Substanzen und Mineralsalzen, stickstoffhaltigen Substanzen und anderen in Wasser unlöslichen oder wenig löslichen Zusatzstoffen herstellt;

b) man eine wäßrige Lösung enthaltend wenigstens einen Bestandteil ausgewählt unter den Aromatisierungsmitteln wie auch den Mineralsalzen, stickstoffhaltigen Stoffen und anderen in Wasser löslichen Zusatzstoffen herstellt;

c) man zu der trocknen homogenen Mischung die wäßrige Lösung unter Kneten zusetzt, um so eine Paste zu erhalten; und

d) man die Paste trocknet.

## Claims

1. Compositions for ingestion by ruminants, characterised in that they contain a peroxide of a metal of group 2 of the Periodic Table.

2. Compositions according to Claim 1, characterised in that they contain magnesium peroxide.

3. Compositions according to Claim 1, characterised in that they contain calcium peroxide.

4. Compositions according to any one of Claims 1 to 3, characterised in that they additionally contain at least one feedstuff for ruminants.

5. Compositions according to Claim 4, characterised in that they contain 0,2 to 5% by weight of peroxide.

6. Compositions according to any one of Claims 1 to 3, characterised in that they additionally contain at least one additive chosen from amongst carbohydrate carriers, vitamins, inorganic salts, trace elements, emulsifiers, flavourings, binders and certain active substances suited to the specific needs of the animal.

7. Compositions according to Claim 6, characterised in that they contain form 10 to 60% by weight of peroxide.

8. Compositions according to any one of Claims 1 to 3, characterised in that they are in the form of a lickstone and furthermore contain at least one additive chosen from amongst vitamins, trace elements, nitrogen-containing non-protein materials, inorganic salts, sulphur-containing materials, carbohydrate carriers, flavourings and the other additives normally used in lickstones.

9. Compositions according to Claim 8, characterised in that they contain from 5 to 50% by weight of peroxide.

10. Process for the preparation of compositions according to Claim 8 or 9, characterised in that:

a) a dry homogeneous mixture is prepared which contains a peroxide of a metal of group 2 of the Periodic Table and at least one other constituent which is insoluble or spraingly soluble in water, chosen from amongst carbohydrate carriers, fats, mixtures based on trace elements and on vitamins, sulphur-containing materials and also inorganic salts, nitrogen-containing materials and other additives which are insoluble or sparingly soluble in water;

b) an aqueous solution is prepared which contains at least one constituent chosen from amongst flavourings and also inorganic salts, nitrogen-containing materials and other additives which are soluble in water;

c) the aqueous solution is added to the dry homogeneous mixture, with kneading, so as to obtain a paste; and

d) the paste is dried.

FIG 1

0 032 409

FIG 2

FIG 3